(19) Europäisches Patentamt
European Patent Office
Office européen des brevets

(11) Veröffentlichungsnummer: **0 008 072**
**B1**

(12) # EUROPÄISCHE PATENTSCHRIFT

(45) Veröffentlichungstag der Patentschrift: **22.02.84**

(21) Anmeldenummer: **79102752.7**

(22) Anmeldetag: **01.08.79**

(51) Int. Cl.³: **C 07 D 295/18, A 61 K 31/635**

(54) **Substituierte Anthranilsäureamide, Verfahren zu ihrer Herstellung, diese enthaltende pharmazeutische Präparate und ihre Herstellung.**

(30) Priorität: **10.08.78 CH 8525/78**

(43) Veröffentlichungstag der Anmeldung:
**20.02.80 Patentblatt 80/4**

(45) Bekanntmachung des Hinweises auf die Patenterteilung:
**22.02.84 Patentblatt 84/8**

(84) Benannte Vertragsstaaten:
**AT BE CH DE FR GB IT LU NL SE**

(56) Entgegenhaltungen:
**DE - A - 2 431 609**
**DE - B - 1 445 629**

(73) Patentinhaber: **CIBA-GEIGY AG**
**Patentabteilung Postfach**
**CH-4002 Basel (CH)**

(72) Erfinder: **Ferrini, Pier Giorgio, Dr.**
**Im Rehwechsel 22**
**CH-4102 Binningen (CH)**
Erfinder: **Rossi, Alberto, Dr.**
**Bündtenweg 30**
**CH-4104 Oberwil (CH)**
Erfinder: **Haas, Georges, Dr.**
**Im Rehwechsel 24**
**CH-4102 Binningen (CH)**

**EP 0 008 072 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Substituierte Anthranilsäureamide, Verfahren zu ihrer Herstellung, diese enthaltende pharmazeutische Präparate und ihre Herstellung

Die Erfindung betrifft neue substituierte Anthranilsäureamide der Formel

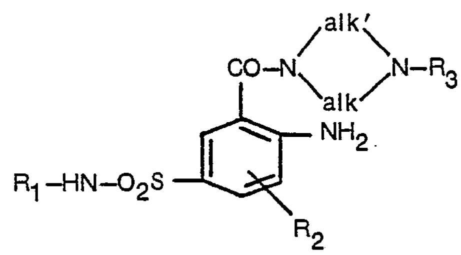

(I),

worin $R_1$ Wasserstoff, Niederalkyl mit bis zu 4 C-Atomen, Niederalkenyl mit bis zu 4 C-Atomen, Cycloalkyl mit 5 bis 8 Ringgliedern, Bicyclo- oder Tricycloalkyl mit jeweils 5 oder 6 Ringgliedern, Cycloalkylniederalkyl mit 5 bis 8 Ringgliedern und mit bis zu 4 C-Atomen im Niederalkylenteil oder Bicyclo- oder Tricycloalkylniederalkyl mit jeweils 5 oder 6 Ringgliedern und mit bis zu 4 C-Atomen im Niederalkylenteil bedeutet, $R_2$ Wasserstoff, Niederalkyl mit bis zu 4 C-Atomen, Niederalkoxy mit bis zu 4 C-Atomen, Halogen bis Atomnummer 35 oder Trifluormethyl bedeutet, alk und alk' gleiches oder verschiedenes, die Stickstoffatome durch 2 C-Atome trennendes Niederalkylen mit bis zu 4 C-Atomen darstellt und $R_3$ Wasserstoff, Niederalkyl mit bis zu 4 C-Atomen, Niederalkenyl mit bis zu 4 C-Atomen, oder einen gegebenenfalls im Phenyl- bzw. Pyridyl- oder Thienylteil durch Niederalkyl mit bis zu 4 C-Atomen, Niederalkoxy mit bis zu 4 C-Atomen, Halogeh bis Atomnummer 35 und/oder Trifluormethyl substituierten und im Niederalk(en)ylenteil bis zu 4 C-Atome aufweisenden Phenylniederalkyl-, Phenylniederalkenyl-, Pyridylniederalkyl- oder Thienylniederalkylrest darstellt und ihre Salze, Verfahren zu ihrer Herstellung, ihre Verwendung als Arzneimittel und diese enthaltende pharmazeutische Präparate.

Cycloalkylreste mit 5 bis 8 Ringgliedern sind beispielsweise Cyclopentyl, Cyclohexyl, Cycloheptyl oder Cyclooctyl. Bicoycloalkylreste, in denen die einzelnen Ringe jeweils 5 oder 6 Ringgliedern aufweisen, sind beispielsweise 2-Bornyl, 2-Norbornyl, 2-Bicyclo[2,2,2]octyl oder 2- oder 3-Bicyclo[4,4,0]decyl. Tricycloalkylreste, in denen die Cycloalkylreste 5 oder 6 Ringglieder aufweisen sind beispielsweise 1- oder 2-Adamantyl.

Gegebenenfalls substituierte Phenylniederalkyl- oder Phenylniederalkenylreste sind beispielsweise im Phenylteil gegebenenfalls durch Niederalkyl, Niederalkoxy, Halogen und/oder Trifluormethyl substituiert.

Gegebenenfalls substituierte Pyridylniederalkyl- oder Thienylniederalkylreste sind beispielsweise gegebenenfalls durch Niederalkyl, Niederalkoxy, Halogen und/oder Trifluormethyl substituiert.

Vor- und nachstehend werden unter "niederen" organischen Resten und Verbindungen vorzugsweise solche, die bis und mit 7, insbesondere bis und mit 4, Kohlenstoffatome (C-Atome) aufweisen, verstanden.

Niederalkyl ist beispielsweise Methyl, Aethyl, Propyl, Isopropyl, n-, sek.-, iso- oder tert.-Butyl, ferner Pentyl, Hexyl oder Heptyl.

Niederalkoxy ist beispielsweise Methoxy, Aethoxy, Propyloxy, Isopropyloxy, n-, sek.-, iso- oder tert.-Butyloxy, ferner Pentyloxy, Hexyloxy oder Heptyloxy.

Halogen ist beispielsweise Halogen bis und mit Atomnummer 35, wie Fluor, Chlor oder Brom.

Niederalkylen, welches die Stickstoffatome in Formel I durch oder 3 Kettenglieder trennt, ist beispielsweise Aethylen oder Propylen-1,3, ferner Propylen-1,2 oder Butylen-1,3 oder -2,3.

Niederalkenyl ist beispielsweise Vinyl, Allyl oder Methallyl.

Phenylniederalkyl ist beispielsweise Benzyl, 1- oder 2-Phenyläthyl, 1-, 2- oder 3-Phenylpropyl, 1- oder 2-Phenylisopropyl oder 3- oder 4-Phenylbutyl.

Phenylniederalkenyl ist beispielsweise 3-Phenyl-propen-2-yl.

Pyridylniederalkyl ist beispielsweise 2-, 3- oder 4-Pyridylmethyl, 1- oder 2-(2-, 3- oder 4-Pyridyl)-äthyl, 1-, 2- oder 3-(2-, 3- oder 4-Pyridyl)-propyl, 1- oder 2-(2-, 3- oder 4-Pyridyl)-isopropyl oder 3- oder 4-(2-, 3- oder 4-Pyridyl)-butyl.

Thienylniederalkyl ist beispielsweise 2- oder 3-Thienylmethyl, 1- oder 2-(2- oder 3-Thienyl)-äthyl, 1-, 2- oder 3-(2- oder 3-Thienyl)-propyl, 1- oder 2-(2- oder 3-Thienyl)-isopropyl oder 3- oder 4-(2- oder 3-Thienyl)-butyl.

Die Verbindungen der Formel I weisen wertvolle pharmakologische Eigenschaften auf. Insbesondere besitzen sie eine ausgeprägte antinociceptive (analgetische) Wirkung, die sich beispielsweise anhand des Essigsäure-Writhing-Syndroms an der Maus im Dosisbereich von etwa 100 bis 300 mg/kg p.o. und an der Ratte im Dosisbereich von etwa 10 bis etwa 100 mg/kg p.o. zeigen lässt. Ferner weisen sie eine deutliche anti-inflammatorische Wirkung auf, die sich z.B. an der Ratte anhand der Terpentin-Pleuritis im Dosisbereich von etwa 10 bis etwa 300 mg/kg p.o. zeigen lässt. Ferner weisen sie eine ausgeprägte antiallergische Wirkung auf, die sich z.B. an der Ratte anhand einer passiven kutanen Anaphylaxie im Dosisbereich von 3 bis etwa 30 mg/kg p.o. zeigt. Die Verbindungen der Formel I können dementsprechend als Analgetika, Antiinflammatorika und/oder Antiallergika verwendet werden.

Die neuen Verbindungen zeigen gegenüber den im Stand der Technik (DE—AS—1 445 629 sowie DE—OS—2 431 609) beschriebenen Verbindungen eine deutlich überlegene pharmakologische, z.B. antiinflammatorische, Aktivität.

Die Erfindung betrifft insbesondere Verbindungen der Formel I, worin $R_1$ Wasserstoff, Niederalkyl mit bis zu 4 C-Atomen, wie Methyl, Aethyl, Propyl, Isopropyl, Butyl, Isobutyl oder Sekundärbutyl, Niederalkenyl mit bis zu 4 C-Atomen, wie Allyl, Cycloalkyl mit 5 bis 8 Ringgliedern, wie Cyclopentyl, Cyclohexyl oder Cycloheptyl, Bicyclo- oder Tricycloalkyl mit jeweils 5 oder 6 Ringgliedern, wie 2-Bornyl, 2-Norbornyl, 2-Bicyclo[2,2,2]octyl oder 1- oder 2-Adamantyl, Cycloalkylniederalkyl mit 5 bis 8 Ringgliedern und mit bis zu 4 C-Atomen im Niederalkylenteil, wie Cyclopentylmethyl, Cyclohexylmethyl oder Cycloheptylmethyl, oder Bicyclo- oder Tricycloalkylniederalkyl mit jeweils 5 oder 6 Ringgliedern und mit bis zu 4 C-Atomen im Niederalkylenteil, wie 2-Bornylmethyl, 2-Norbornylmethyl oder 2-Bicyclo[2,2,2]octylmethyl, bedeutet, $R_2$ Wasserstoff, Niederalkyl mit bis zu 4 C-Atomen, wie Methyl, Niederalkoxy mit bis zu 4 C-Atomen, wie Methoxy, Halogen bis Atomnummer 35, wie Chlor, oder Trifluormethyl bedeutet, alk und alk' gleiches oder verschiedenes, die Stickstoffatome durch 2 C-Atome trennendes Niederalkylen mit bis zu 4 C-Atomen, wie Aethylen, 1,2-Propylen oder 2,3-Butylen, darstellt und $R_3$ Wasserstoff, Niederalkyl mit bis zu 4 C-Atomen, wie Methyl, Niederalkenyl mit bis zu 4 C-Atomen, wie Allyl, oder einen gegebenenfalls im Phenyl- bzw. Pyridyl- oder Thienylteil durch Niederalkyl mit bis zu 4 C-Atomen, wie Methyl, Niederalkoxy mit bis zu 4 C-Atomen, wie Methoxy, Halogen bis Atomnummer 35, wie Chlor, und/oder Trifluormethyl substituierten und im Niederalk(en)ylenteil bis zu 4 C-Atome aufweisenden Phenylniederalkyl-, wie Benzyl-, 2-Phenyläthyl oder 3-Phenylpropylrest, Phenylniederalkenyl-, wie 3-Phenyl-propen-2-ylrest, Pyridylniederalkyl-, wie 2-, 3- oder 4-Pyridylmethyl-, 2-(2-, 3- oder 4-Pyridyl)-äthyl- oder 3-(2-, 3- oder 4-Pyridyl)-propylrest oder Thienylniederalkyl-, wie 2- oder 3-Thienylmethyl-, 2-(2- oder 3-Thienyl)-äthyl- oder 3-(2- oder 3-Thienyl)-propylrest darstellt, und ihre Salze.

Die Erfindung betrifft vor allem Verbindungen der Formel I, worin $R_1$ Wasserstoff, Niederalkyl mit bis zu 4 C-Atomen, wie Methyl, Aethyl, Propyl, Isopropyl, Butyl, Isobutyl oder Sekundärbutyl, Cycloalkyl mit 5 bis 8 Ringgliedern, wie Cyclopentyl, Cyclohexyl oder Cycloheptyl, oder Tricycloalkyl mit jeweils 5 oder 6 Ringgliedern, wie 1- oder 2-Adamantyl, bedeutet, $R_2$ Wasserstoff, Niederalkyl mit bis zu 4 C-Atomen, wie Methyl, Niederalkoxy mit bis zu 4 C-Atomen, wie Methoxy, Halogen bis Atomnummer 35, wie Chlor, oder Trifluormethyl bedeutet, alk und alk' gleiches, die Stickstoffatome durch 2 C-Atome trennendes Niederalkylen mit bis zu 4 C-Atomen, wie Aethylen, 1,2-Propylen oder 2,3-Butylen, darstellt und $R_3$ Wasserstoff, Niederalkyl mit bis zu 4 C-Atomen, wie Methyl, oder einen gegebenenfalls im Phenylteil durch Niederalkyl mit bis zu 4-C-Atomen, wie Methyl, Niederalkoxy mit bis zu 4 C-Atomen, wie Methoxy, Halogen bis Atomnummer 35, wie Chlor, und/oder Trifluormethyl substituierten, und im Niederalkylenteil bis zu 4 C-Atome aufweisenden Phenylniederalkyl-, wie Benzyl-, 2-Phenyläthyl oder 3-Phenylpropylrest darstellt, und ihre Salze.

Die Erfindung betrifft in allererster Linie Verbindungen der Formel I, worin $R_1$ Niederalkyl mit bis zu 4 C-Atomen, wie Methyl, Aethyl, Propyl, Isopropyl, Butyl, Sekundärbutyl oder Isobutyl, bedeutet, $R_2$ Wasserstoff ist, alk und alk' Aethylen bedeuten und $R_3$ Wasserstoff, Niederalkyl mit bis zu 4 C-Atomen, wie Methyl, oder einen im Phenylteil gegebenenfalls durch Niederalkyl mit bis zu 4 C-Atomen, wie Methyl, Niederalkoxy mit bis zu 4 C-Atomen, wie Methoxy, und/oder Halogen substituierten, bis zu 10 C-Atome aufweisenden Phenylniederalkyl-, wie Benzyl- oder 2-Phenyläthylrest, bedeutet, und ihre Salze.

Die Erfindung betrifft namentlich die in den Beispielen genannten Verbindungen der Formel I und ihre Salze.

Die Verbindungen der Formel I können nach an sich bekannten Methoden hergestellt werden.

Eine bevorzugte Verfahrensweise ist dadurch gekennzeichnet, dass man in einer Verbindung der Formel

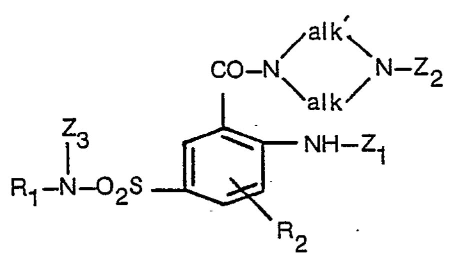

(II)

worin mindestens einer der Reste $Z_1$, $Z_2$ und $Z_3$ einen durch Wasserstoff ersetzbaren Rest Z und ein gegebenenfalls von Z verschiedener Rest $Z_1$, $Z_2$ bzw. $Z_3$ für Wasserstoff oder im Falle eines Restes $Z_2$ für

eine von Wasserstoff verschiedene Gruppe $R_3$ steht, den Rest Z durch Wasserstoff ersetzt und gewünschtenfalls die so erhältliche Verbindung in eine andere Verbindung der Formel I umwandelt und/oder eine erhaltene freie Verbindung in ein Salz oder ein erhaltenes Salz in die freie Verbindung oder in ein anderes Salz überführt.

Durch Wasserstoff ersetzbare Reste Z sind beispielsweise reduktiv oder solvolytisch durch Wasserstoff ersetzbare Reste.

Reduktiv durch Wasserstoff ersetzbare Reste sind beispielsweise gegebenenfalls substituierte Benzyloxycarbonylreste, z.B. Benzyloxy- oder p-Nitrobenzyloxycarbonyl, oder 2-Halogenniederalkoxycarbonylreste, z.B. 2-Jodäthoxy-, 2,2,2-Trichloräthoxy- oder 2,2,2-Trichlor-tert.-butyloxycarbonyl. Weitere durch Wasserstoff ersetzbare Reste $Z_1$ sind Hydroxy, gegebenenfalls durch einen aromatischen Rest, wie gegebenenfalls substituiertes Phenyl, substituiertes Amino, z.B. Anilino, und weitere ersetzbare Reste $Z_1$ bzw. $Z_3$ sind $\alpha$-Aralkylreste, z.B. Benzyl.

Der reduktive Ersatz der genannten Reste durch Wasserstoff erfolgt in üblicher Weise, beispielsweise durch Behandlung mit Wasserstoff in Gegenwart eines Hydrierungskatalysators, wie eines Nickel-, Platin-, Rhodium-, Ruthenium- oder Palladiumkatalysators, z.B. von Raney-Nickel, Platin oder Platinoxid, gegebenenfalls auf Kohle, einem Komplex von Rhodiumchlorid oder Rutheniumchlorid und einem Phosphin, wie Triphenyl- oder Triäthylphosphin, oder von Palladium auf Kohle, oder ausgehend von Verbindungen der Formel II, worin $Z_1$ Hydroxy ist, eines Kupferkatalysators, z.B. von Kupfer-I-oxid. Vorteilhaft arbeitet man in einem inerten Lösungsmittel, wie einem Niederalkanol, z.B. in Methanol oder Aethanol, oder einer Niederalkansäure, wie Essigsäure, erforderlichenfalls bei erhöhtem Druck, bei erhöhter Temperatur und/oder im geschlossenen Gefäss. 2-Halogenniederalkoxycarbonyl und/oder Hydroxy $Z_1$ kann ferner durch Umsetzung mit einem unedlen Metall, wie Zink, in Essigsäure, oder mit einer niederwertigen Uebergangsmetallverbindung, wie Kobalt-II-chlorid, Hydroxygruppen $Z_1$ auch durch Umsetzung mit einem geeigneten Dileichtmetallhydrid, z.B. mit Lithiumborhydrid oder sulfuriertem Natriumborhydrid, durch Wasserstoff ersetzt werden.

Solvolytisch durch Wasserstoff ersetzbare Reste Z sind beispielsweise Acylreste, wie von gegebenenfalls halogenierten Niederalkansäuren oder der gegebenenfalls substituierten Benzoesäure abgeleitete Acylreste, z.B. Formyl, Acetyl, Trifluoracetyl oder Benzoyl, von monofunktionellen Derivaten der Kohlensäure abgeleitete Acylreste, wie gegebenenfalls durch Halogen, Niederalkansulfonyl oder Niederalkylthio, gegebenenfalls substituiertes Benzolsulfonyl oder Phenylthio, oder Silylreste substituierte Niederalkoxycarbonylreste, z.B. Aethoxycarbonyl, 2-Jodäthoxy- oder 2,2,2-Trichlor-tert.-butoxycarbonyl, 2-Niederalkansulfonyl-, 2-Niederalkylthio- oder 2-Trimethylsilyl-äthoxycarbonyl, gegebenenfalls substituiertes 2-(Benzolsulfonyl)- oder 2-(Phenylthio)-äthoxycarbonyl, beispielsweise 2-(p-Toluolsulfonyl)- oder 2-(p-Methylphenylthio)-äthoxycarbonyl, gegebenenfalls substituierte Benzyloxycarbonylreste, z.B. Benzyl- oder p-Nitrobenzyloxycarbonyl, Cycloalkoxycarbonylreste, z.B. Isobornyloxycarbonyl, oder Halogencarbonyl, z.B. Chlorcarbonyl, ferner Cyano oder Silylreste, wie Trimethylsilyl. Weitere solvolytisch durch Wasserstoff ersetzbare Reste $Z_1$ sind in $\alpha$-Stellung hydroxysubstituierte, gegebenenfalls weiter substituierte Kohlenwasserstoffreste, wie $\alpha$-Hydroxyniederalkyl, z.B. 1-Hydroxyäthyl oder 2-(2-Hydroxy)-propyl, $\alpha$-Hydroxycycloalkylreste, wie 1-Hydroxycyclohexyl oder gegebenenfalls substituierte $\alpha$-Hydroxy-$\alpha$-phenyl-niederalkyl-, wie -benzylreste.

Der solvolytische Ersatz der genannten solvolysierbaren Gruppen Z durch Wasserstoff erfolgt in üblicher Weise, beispielsweise durch Hydrolyse, erforderlichenfalls in Gegenwart eines sauren oder basischen Hydrolysemittels und/oder eines, vorzugsweise mit Wasser mischbaren, Lösungsmittels, wie eines Niederalkanols, z.B. von Aethanol, eines Diniederalkylketons, z.B. von Aceton, oder eines Niederalkansäureamides, z.B. von Dimethylformamid, und/oder bei erhöhter Temperatur. Saure Hydrolysemittel sind beispielsweise Protonensäuren, wie Mineralsäuren, z.B. Salzsäure, Schwefelsäure oder Phosphorsäure, oder organische Carbonsäuren, wie gegebenenfalls halogenierte Niederalkansäuren, z.B. Ameisensäure, Essigsäure oder Chlor- oder Trichloressigsäure. Basische Hydrolysemittel sind beispielsweise Alkalimetall- oder Erdalkalimetallhydroxide, wie Natrium-, Kalium- oder Calciumhydroxid, Alkalimetallcarbonate, wie Natrium- oder Kaliumcarbonat, Ammoniak oder organische Stickstoffbasen, wie Tribenzyl-methyl-ammoniumhydroxid. Tertiäre Niederalkoxycarbonylgruppen, wie tert.-Butoxycarbonyl, können ferner durch Umsetzung mit einer geeigneten Carbonsäure, wie Trifluoressigsäure, Silyl- under 2-Silyläthoxycarbonylgruppen durch Umsetzung mit einem Salz der Fluorwasserstoffsäure, z.B. mit Kaliumfluorid in Acetonitril, unter wasserfreien Bedingungen solvolytisch durch Wasserstoff ersetzt werden.

Die Ausgangsstoffe der Formel II können nach an sich bekannten Methoden hergestellt werden, beispielsweise indem man eine Säure der Formel

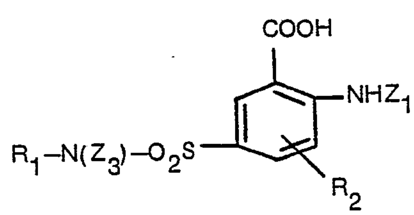

(IIa)

oder ein rekationsfähiges funktionelles Derivat davon mit einem Amin der Formel

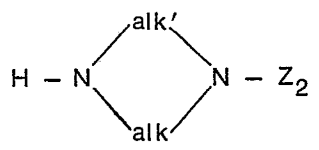

(IIb)

oder einem reaktionsfähigen Derivat oder Salz davon, z.B. eine 5-($R_1$-Sulfamoyl)-N-$Z_1$-anthranilsäure mit dem N-Chlorcarbonyl- oder einem N-Niederalkan-, wie N-Methan-, oder N-Benzolsulfensäurederivat einer Verbindung der Formel IIb oder ein 5-($R_1$-Sulfamoyl)-isatosäureanhydrid mit einer Verbindung der Formel IIb, in der $Z_2$ einen Rest Z bedeutet, umsetzt. Die Umsetzung erfolgt in üblicher Weise, insbesondere wie nachstehend für die Umsetzung von Verbindungen der Formeln III und IV bzw. ihrer reaktionsfähigen Derivate angegeben.

Ausgangsstoffe der Formel II, worin $Z_1$ Hydroxy als redkutiv durch Wasserstoff ersetzbaren Rest bedeutet, werden vorteilhaft *in situ* unter den Reduktionsbedingungen durch Reduktion aus den entsprechenden 5-($R_1$-Sulfamoyl)-2-nitro-benzoesäureamiden oder ihren Salzen hergestellt. Diese wiederum sind durch Umsetzung entsprechender 5-(Chlorsulfonyl)-2-nitrobenzoesäuren mit einer Verbindung der Formel $R_1$—$NH_2$, gegebenenfalls Ueberführung der so erhältlichen 5-($R_1$-Sulfamoyl)-2-nitrobenzoesäure in ein reaktives Derivat, z.B. mit Thionylchlorid in das Säurechlorid, und nachfolgende übliche Kondensation mit dem Amin der Formel IIb oder einem reaktiven Derivat bzw. Salz davon zugänglich. Analog werden Verbindungen der Formel II, in denen $Z_1$ einen in $\alpha$-Stellung durch Hydroxy substituierten Kohlenwasserstoffrest bedeutet, vorteilhaft durch saure Hydrolyse der entsprechenden, am Anilino-N-atom durch einen zweiwertigen Kohlenwasserstoffrest, der substituiert sein kann, substituierten 5-($R_1$-Sulfamoyl)-anthranilsäureamide *in situ* hergestellt. Diese erhält man z.B. durch Umsetzung der entsprechenden 5-Chlorsulfonyl-2-chlor-benzoesäuren mit einem Amin $R_1$—$NH_2$, anschliessende Ammonolyse, Kondensation der so erhältlichen 5-($R_1$-Sulfamoyl)-anthranilsäure mit $\alpha$-Oxokohlenwasserstoffen, z.B. Benzaldehyd oder Acetaldehyd, Ueberführung des Produktes in das Chlorid und Umsetzung des so erhältlichen am Anilino-N-atom substituierten 5-($R_1$-Sulfamoyl)-anthraniloylchlorides, z.B. 5-($R_1$-Sulfamoyl)-2-benzylidenamino-benzoylchlorid, mit einer Verbindung der Formel IIb.

Die Verbindungen der Formel I können ferner hergestellt werden, indem man eine Säure der Formel

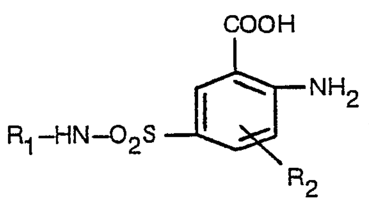

(III)

oder ein reaktionsfähiges funktionelles Derivat davon mit einem Amin der Formel

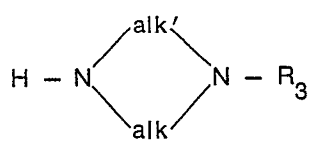

(IV)

oder einem reaktionsfähigen Derivat oder Salz davon umsetzt und gewünschtenfalls die so erhältliche Verbindung in eine andere Verbindung der Formel I umwandelt und/oder eine erhaltene freie Verbindung in ein Salz oder ein erhaltenes Salz in die freie Verbindung oder in ein anderes Salz überführt.

Reaktionsfähige funktionelle Derivate von Säuren der Formel III sind beispielsweise reaktionsfähige Ester, wie gegebenenfalls substituierte Phenylester, z.B. Phenyl-, p-Nitrophenyl- oder 2,4-Dinitrophenylester, reaktionsfähige Amide, wie 1-Imidazolide, oder Anhydride derselben, wie offenkettige Anhydride mit Mineralsäure, z.B. Säurechloride, Anhydride mit phosphinigen oder phosphorigen Säuren, z.B. mit Diphenylphosphiniger Säure, mit Kohlensäurehalbestern oder sauren Phosphorigsäureestern, wie Kohlensäuremonophenyl- oder Kohlensäuremonoäthylester oder Phosphorigsäurediäthylester, oder cyclische Anhydride davon, wie Verbindungen der Formel

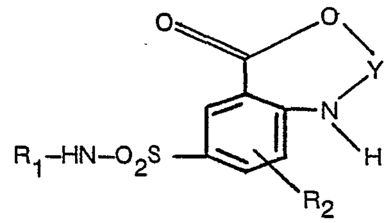

(IIIa),

worin Y eine Carbonyl-, Thiocarbonyl- oder Sulfinylgruppe oder eine Gruppe der Formeln >P—R, >P(=O)—R oder >P(=O) ($R_2$), in der R einen organischen Rest, wie Niederalkyl, z.B. Methyl, oder gegebenenfalls substituiertes Phenyl bedeutet, darstellt.

Reaktionsfähige Derivate von Verbindungen der Formel IV sind beispielsweise deren von Halbestern der Kohlensäure oder Halogenameisensäure abgeleitete Acylderivate, wie Niederalkoxycarbonyl- oder Halogen-, z.B. Chlorcarbonylderivate, oder Sulfenylamide derselben mit organischen Sulfensäuren, wie Niederalkansulfensäuren, oder gegebenenfalls substituierten Benzolsulfensäuren, z.B. mit Methansulfensäure oder Benzolsulfensäure.

Die Umsetzung von Verbindungen der Formeln III und IV bzw. von reaktionsfähigen Derivaten derselben erfolgt in der jeweils üblichen Weise.

Bei der Umsetzung von Säuren der Formel III mit Aminen der Formel IV oder deren Salzen arbeitet man vorteilhaft in Gegenwart eines wasserbindenden Mittels, vorzugsweise von Phosphorpentoxid oder einem Ester der pyrophosphorigen Säure, z.B. von Tetraäthylpyrophosphit, oder unter destillativer, vorzugsweise azeotrop-destillativer Entfernung des Reaktionswassers, erforderlichenfalls in einem inerten Lösungsmittel, wie Toluol, und/oder bei erhöhter Temperatur, z.B. bei etwa 50 bis 200°C.

Die Umsetzung von reaktionsfähigen Estern oder Amiden oder von Anhydriden von Säuren der Formel III mit Aminen der Formel IV oder deren Salzen wird vorteilhaft in einem gegenüber den Reaktionsteilnehmern inerten Lösungsmittel, z.B. in Toluol, Xylol, Tetrahydrofuran oder Dioxan, erforderlichenfalls in Gegenwart eines basischen Kondensationsmittels, wie einer tertiären organischen Stickstoffbase, wie Triäthylamin oder Pyridin, und/oder bei erniedrigter oder erhöhter Temperatur, z.B. im Temperaturbereich von etwa 0° bis etwa 150°, durchgeführt.

Die Umsetzung von Säuren der Formel III mit Acylderivaten von Aminen der Formel IV erfolgt vorzugsweise unter Erhitzen, z.B. bei etwa 100 bis 250°C, erforderlichenfalls in einem gegenüber den Reaktionsteilnehmern inerten Lösungsmittel, wie Xylol, während man die Umsetzung mit von Aminen der Formel IV abgeleiteten Sulfenylamiden vorzugsweise bei normaler Temperatur, z.B. bei etwa 0 bis 50°C, vorzugsweise in einem inerten Lösungsmittel, wie einem N,N-Diniederalkylamid, z.B. Dimethylformamid, oder N-Methylpyrrolidon, in Pyridin, in einem Aether, z.B. Diäthyläther, Dioxan oder Tetrahydrofuran, in Benzol, Toluol oder Xylol, durchführt.

Die Ausgangsstoffe der Formel III und IV können in an sich bekannter Weise hergestellt werden.

Säuren der Formel III werden beispielsweise erhalten, indem man eine entsprechende 5-Chlorsulfonyl-2-chlor-benzoesäure mit einem Amin $R_1$—$NH_2$ umsetzt und die erhaltene 5-($R_1$-Sulfamoyl)-2-chlor-benzoesäure in üblicher Weise, ammonolysiert.

Reaktionsfähige Ester von Säuren der Formel III werden beispielsweise durch übliche Veresterung, wie Umsetzung mit dem entsprechenden Alkohol in Gegenwart einer Mineralsäure, wie Schwefelsäure oder Chlorwasserstoffsäure erhalten.

Reaktionsfähige Amide von Säuren der Formel III können beispielsweise durch Umsetzung der Säure in üblicher Weise mit einem entsprechenden Harnstoff, wie Bis-(1-Imidazolyl)-harnstoff, mit einem Dihalogenid, Diester oder Esterhalogenid der Kohlensäure, wie einem Halogenameisensäureniederalkylester, mit einem Ester oder Halogenid einer phosphinigen Säure der Formel $(R)_2P$—OH, wie einem Benzolphosphinigsäureniederalkylester, z.B. -äthylester, oder Benzolphosphinigsäurechlorid, einem Diester, Esterhalogenid oder Dihalogenid einer phosphonigen Säure der Formel R—$P(OH)_2$, z.B. Benzolphosphonigsäuredichlorid, oder einem Diester, Esterhalogenid oder Halogenid einer Phosphonsäure der Formel R—P( = O) $(OH)_2$, z.B. mit Benzolphosphonsäuredichlorid, erhalten werden. Isatosäuredichloride, der Formel III, worin Y Carbonyl ist, werden vorteilhaft hergestellt, indem man 5-Chlorsulfonylisatosäure in üblicher Weise mit einem Amin der Formel $R_1$—$NH_2$ umsetzt.

Offenkettige Anhydride von Säuren der Formel III werden vorteilhaft unter den Reaktionsbedingungen *in situ* gebildet, indem man die entsprechende Säure beispielsweise mit einem Halogenierungsmittel, wie Thionylchlorid, oder mit einem Ester oder Halogenid einer phosphinigen Säure oder einem Ester, Esterhalogenid oder Dihalogenid einer phosphonigen Säure oder Phosphonsäure, z.B. mit Benzolphosphinigsäurechlorid, Benzolphosphinigsäureäthylester oder Benzolphosphonigsäure- bzw. Benzolphosphonsäuredichlorid-, äthylesterchlorid oder -diäthylester, umsetzt.

Reaktionsfähige Derivate von Aminen der Formel IV werden ebenfalls vorteilhaft unter den Reaktionsbedingungen *in situ* erzeugt, indem man das Amin mit einem Halogenameisensäureester, oder in Gegenwart einer trivalenten Phosphorverbindungen, z.B. von Triphenylphosphin, mit einem organischen Disulfid, z.B. einem Diniederalkyldisulfid, umsetzt.

Erfindungsgemäss erhältliche Verbindungen können in andere Verbindungen der Formel I umgewandelt werden.

So kann man in Verbindungen der Formel I, in denen $R_3$ Wasserstoff bedeutet, einen von Wasserstoff verschiedenen Rest $R_3$ einführen. Die Einführung des Restes $R_3$ erfolgt in üblicher Weise, beispielsweise durch Umsetzung mit einem reaktionsfähigen Ester eines aliphatischen oder gegebenenfalls substituierten araliphatischen oder heteroarylaliphatischen Alkohols, wie einem Ester desselben mit einer organischen Sulfonsäure, wie mit Benzol-, p-Brombenzol-, Toluol- oder Methansulfonsäure, oder mit einer anorganischen Sulfonsäure, wie mit Fluorsulfonsäure, oder mit einer Mineralsäure, wie mit Chlor-, Brom- oder Jodwasserstoffsäure oder Schwefelsäure.

Die Umsetzung erfolgt vorzugsweise in Gegenwart eines säurebindenden Mittels, wie einer tertiären organischen Base, z.B. von Aethyldiisopropylamin, Pyridin oder Chinolin, oder einer anorganischen Base, wie eines Alkalimetall- oder Erdalkalimetallhydroxides oder eines Alkalimetallcarbonates, z.B. von Natrium-, Kalium- oder Calciumhydroxid oder Kalium- oder Natriumcarbonat. Vorzugsweise werden die beschriebenen Umsetzungen in einem inerten organischen Lösungsmittel, wie einem Alkanol, z.B. in Aethanol oder Methanol, oder einem Kohlenwasserstoff, z.B. in Benzol, Toluol oder Xylol, einem Aether, wie Diäthyläther, Tetrahydrofuran oder Dioxan oder einem chlorierten oder nitrierten Kohlenwasserstoff, z.B. in Methylenchlorid, Tetrachlormethan, Nitrobenzol oder Nitromethan durchgeführt.

Die Einführung eines Restes $R_3$ kann aber auch durch Umsetzung mit einer entsprechenden Oxo-Verbindung, z.B. mit einem entsprechenden Aldehyd oder Keton unter reduzierenden Bedingungen, z.B. in Gegenwart von Wasserstoff bei Anwesenheit eines Hydrierungskatalysators, wie. eines Palladium-, Platin- oder Nickelkatalysators, z.B. von Palladium auf Kohle oder Calciumcarbonat, von Platinoxid oder Raney-Nickel, unter normalem oder vorzugsweise erhöhtem Druck, oder in Gegenwart eines organischen Reduktionsmittels, wie von Ameisensäure, oder Formaldehyd, gegebenenfalls in wässriger Lösung, wobei man vorzugsweise bei erhöhter Temperatur arbeitet, oder eines Leichtmetallhydrides, z.B. von Diisoamylborhydrid, oder eines Dileichtmetallhydrides wie Natriumcyanoborhydrid oder Natriumborhydrid, jeweils vorteilhaft in einem unter den Reaktionsbedingungen inerten Lösungsmittel, durchgeführt werden.

Umgekehrt kann man in Verbindungen der Formel I, worin $R_3$ von Wasserstoff verschieden ist, $R_3$ durch Wasserstoff ersetzen, beispielsweise durch Umsetzung mit einem Halogenameisensäureester, wie einem Chlor- oder Bromameisensäureniederalkylester, oder einem Halogencyan, wie Brom- oder Chlorcyan, unter Bildung des entsprechenden Urethans bzw. Cyanamides und anschliessende Hydrolyse desselben. $\alpha$-Aralkylreste und $\alpha$-Heteroarylalkylreste $R_3$ können ferner reduktiv, z.B. durch Behandlung mit Wasserstoff in Gegenwart eines Hydrierungskatalysators, wie von Palladium auf Kohle, durch Wasserstoff ersetzt werden.

Umgekehrt kann man in Verbindungen der Formel I, worin $R_3$ von Wasserstoff verschieden ist, $R_3$ durch Wasserstoff ersetzen, beispielsweise durch Umsetzung mit einem Halogenameisensäureester, wie einem Chlor- oder Broameisensäureniederalkylester, oder einem Halogencyan, wie Brom- oder Chlorcyan, unter Bildung des entsprechenden Urethans bzw. Cyanamides und anschliessende Hydrolyse desselben. $\alpha$-Aralkylreste und $\alpha$-Heteroarylalkylreste $R_3$ können ferner reduktiv, z.B. durch Behandlung mit Wasserstoff in Gegenwart eines Hydrierungskatalysators, wie von Palladium auf Kohle, durch Wasserstoff ersetzt werden.

Ferner kann man in Verbindungen der Formel I, in denen $R_2$ Wasserstoff bedeutet, von Wasserstoff verschiedene Reste $R_2$ einführen. So kann man in üblicher Weise Halogen einführen, beispielsweise durch Umsetzung mit Chlor oder Brom, vorzugsweise in Gegenwart eines Katalysators, wie von Eisen-(III)-chlorid, oder mit N-Chlorsuccinimid. Ferner kann man in üblicher Weise Trifluormethyl einführen, beispielsweise durch Umsetzung mit Trifluorjodmethan in Gegenwart von Metallen, wie Kupferpulver. Weiterhin kann in üblicher Weise Niederalkyl eingeführt werden, beispielsweise durch Umsetzung mit einem Niederalkylhalogenid, vorteilhaft in Gegenwart eines Katalysators, wie eines Metallhalogenides, z.B. von Aluminiumchlorid oder -bromid.

Die genannten Reaktionen können gegebenenfalls gleichzeitig oder nacheinander und in beliebiger Reihenfolge durchgeführt werden.

Die genannten Reaktionen werden in üblicher Weise in An- oder Abwesenheit von Verdünnungs-, Kondensations- und/oder katalytischen Mitteln, bei erniedrigter, gewöhnlicher oder erhöhter Temperatur, gegebenenfalls im geschlossenen Gefäss durchgeführt.

Je nach den Verfahrensbedingungen und Ausgangsstoffen erhält man die Endstoffe in freier Form oder in der ebenfalls in der Erfindung inbegriffenen Form ihrer Säureadditionssalze.

Bei diesen handelt es sich insbesondere um pharmazeutisch verwendbare Säureadditionssalze, wie entsprechende Mineralsäuresalze, z.B. Hydrochloride, Hydrobromide oder Hydrojodide, Sulfate, Hydrogensulfate oder Phosphate, oder entsprechende Carbonsäuresalze, wie Fumarate, Maleinate, Maleate, Citrate, Anthranilate, p-Hydroxybenzoate, Pyruvate, oder Salicylate, ferner entsprechende Sulfonsäuresalze, wie Cyclohexylaminsulfonate, Sulfanilate oder p-Toluolsulfonate. So können beispielsweise basische, neutrale oder gemischte Salze, gegebenenfalls auch Hemi-, Mono-, Sesqui- oder Polyhydrate davon, erhalten werden. Die Säureadditionssalze der neuen Verbindungen können in an sich bekannter Weise in die freie Verbindung übergeführt werden, z.B. mit basischen Mitteln, wie

Alkalien oder Ionenaustauschern. Andererseits können die erhaltenen freien Basen mit organischen oder anorganische Säuren Salze bilden.

Die Salze der neuen Verbindungen, wie z.B. die Pikrate, können auch zur Reinigung der erhaltenen freien Basen dienen, indem man die freien Basen in Salze überführt, diese abtrennt und aus den Salzen wiederum die Basen frei macht. Infolge der engen Beziehungen zwischen den neuen Verbindungen in freier Form und in Form ihrer Salze sind im Vorausgegangenen und nachfolgend unter den freien Verbindungen sinn- und zweckmässig gegebenenfalls auch die entsprechenden Salze zu verstehen.

Die Erfindung betrifft auch diejenigen Ausführungsformen des Verfahrens, bei denen man von einer auf beliebiger Verfahrensstufe als Zwischenprodukte erhältlichen Verbindung ausgeht und die fehlenden Verfahrensschritte ausführt, oder wobei ein Ausgangsstoff unter den Reaktionsbedingungen gebildet oder in Form eines Derivates davon, gegebenenfalls eines Salzes, verwendet wird.

Beim Verfahren der vorliegenden Erfindung werden vorzugsweise solche Ausgangsstoffe verwendet, welche zu den eingangs als besonders wertvoll geschilderten Verbindungen führen.

Bei den erfindungsgemässen pharmazeutischen Präparaten, welche Verbindungen der Formel I oder pharmazeutisch verwendbare Salze davon enthalten, handelt es sich um solche zur enteralen wie oralen oder rektalen, und parenteralen Verabreichung sowie zur topischen Anwendung an Warmblüter(n), welche den pharmakologischen Wirkstoff allein oder zusammen mit einem pharmazeutisch anwendbaren Trägermaterial enthalten. Die Dosierung des Wirkstoffs hängt von der Warmblüter-Spezies, dem Alter und dem individuellen Zustand, sowie von der Applikationsweise ab. Im Normalfall ist für einen etwa 75 kg schweren Warmblüter bei oraler Applikation eine ungefähre Tagesdosis von etwa 15—300 mg, vorteilhaft in mehreren gleichen Teildosen vorteilt, zu veranschlagen.

Die neuen pharmazeutischen Präparate enthalten z.B. von 10% bis 80%, vorzugsweise von 20% bis 60% des Wirkstoffs, Erfindungsgemässe pharmazeutische Präparate zur enteralen bzw. parenteralen Verabreichung sind z.B. solche Dosiseinheitsformen wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Geignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister z.B. von Mais-, Weizen-, Reis- oder Kartoffelstärke-Kleister, Gelatine, Traganth, Methylcellulose und/oder Polyvinylpyrrolidon, und/oder, wenn erwünscht, Sprengmittel, wie die obgenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaft-resistenten Ueberzügen versehen, wobei man u.a. konzentrierte Zuckerlösungen, welche gegebenenfalls arabischen Gummi, Talk, Polyvinylpyrolidon, Polyäthylenglycol und/oder Titandioxid enthalten, Lacklösungen in geeigneten organischen Lösungsmitteln oder Lösungsmittelgemischen oder, zur Herstellung von Magensaftresistenten Ueberzügen, Lösungen von geeigneten Celulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyäthylenglycolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglycole oder höhere Alkanole. Ferner können auch Gelatine-Rektalkapseln verwendet werden, die eine Kombination des Wirkstoffs mit einer Grundmasse enthalten; als Grundmassenstoffe kommen z.B. flüssige Triglyceride, Polyäthylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat oder Triglyceride,

verwendet, oder wässrige Injektionssuspensionen, welche viskositätserhöhende Stoffe, z.B. Natriumcarboxymethylcellulose, Sorbit und/oder Dextran und gegebenenfalls auch Stabilisatoren enthalten.

Als topisch anwendbare pharmazeutische Präparate kommen in erster Linie Creme, Salben, Pasten, Schäume, Tinkturen und Lösungen, in Frage, die von 0,5 bis 20% des Wirkstoffs enthalten.

Die vorliegende Erfindung betrifft ebenfalls die Verwendung der Verbindungen der Formel I und der Salze von solchen Verbindungen mit salzbildenden Eigenschaften, vorzugsweise zur Behandlung von Entzündungen, in erster Linie von entzündlichen chronischen Erkrankungen des rheumatischen Formenkreises, besonders der chronischen Arthritis.

Die nachfolgenden Beispiele illustrieren die oben beschriebene Erfindung; sie sollen jedoch diese in ihrem Umfang in keiner Weise einschränken. Temperaturen sind in Celsiusgraden angegeben.

Beispiel 1

Zu einer Suspension von 10,2 g 5-[N-(1-Adamantyl)-sulfamoyl]-isatosäureanhydrid in 250 ml absolutem Toluol fügt man unter Rühren bei 100° langsam 2,7 g 1-Methylpiperazin. Nach beendeter Zugabe lässt man 1 Stunde bei 100° nachrühren, lässt auf Raumtemperatur abkühlen, dekantiert den Niederschlag ab und kristallisiert das so erhaltene Rohprodukt aus Aethanol um. Man erhält das 5-[N-(1-Adamantyl)-sulfamoyl]-anthranilsäure-(4-methyl)-piperazid vom Smp. 214—215° (Smp. des Hydrochlorides: 298—300°).

Das Ausgangsmaterial kann z.B. wie folgt erhalten werden:

Zu 165 ml Wasser und 36,1 g 1-Amino-adamantan fügt man unter Rühren bei Raumtemperatur portionsweise 30,5 g 2-Chlor-5-chlorosulfonyl-benzoesäure hinzu, lässt 1 Stunde nachrühren, säuert mit konzentrierter Salzsäure auf pH 1 an und filtriert. Man erhält so die 5-[N-(1-Adamantyl)-sulfamoyl]-2-chlor-benzoesäure vom Smp. 207—210°.

Zu 16,9 g dieser Verbindung fügt man 350 ml 25%-ige wässrige Ammoniaklösung und 1 g Kupferpulver hinzu und erwärmt im Autoklaven 12 Stunden auf 125—130°. Dann kühlt man auf Raumtemperatur ab, filtriert durch Kieselgel, säuert mit konzentrierter Salzsäure auf pH 5 an, lässt über Nacht bei 0° stehen und filtriert die gebildeten Kristalle ab. Man erhält so die 5-[N-(1-Adamantyl)-sulfamoyl]-anthranilsäure vom F. 168—171° (Zers.).

Eine Suspension von 14,2 g der obigen Verbindung in 140 ml Essigsäure wird unter Rühren 10 Minuten auf 70° erwärmt. Dann kühlt man auf Raumtemperatur ab und leitet bis zur Sättigung Phosgen ein. Dabei sind die üblichen Sicherheitsmassnahmen zu ergreifen. Anschliessend wird 20 Minuten auf 100° erwärmt. Dann kühlt man auf Raumtemperatur ab, versetzt mit wenig Wasser, nutscht den alsbald ausgefallenden Niederschlag ab, wäscht nacheinander mit Wasser und Diäthyläther und trocknet das kristalline Produkt im Vakuum bei 100°. Man erhält so das 5 - [N - (1 - Adamantyl)-sulfamoyl] - isatosäureanhydrid vom Smp. 164° (Zers.).

Beispiel 2

In analoger Weise wie in Beispiel 1 beschrieben erhält man durch Umsetzung von 5 - [N - (2 - Butyl) - sulfamoyl] - isatosäureanhydrid mit 1-Methylpiperazin das 5 - [N - (2 - Butyl) - sulfamoyl] - anthranilsäure - (4 - methyl) - piperazid vom Smp. 130—132° (aus einem Aethanol/Diäthyläthergemisch). Das Hydrochlorid schmilzt bei 246—248°.

Das als Ausgangsmaterial zu verwendende 5 - [N - (2 - Butyl) - sulfamoyl] - isatosäureanhydrid kann z.B. in analoger Weise wie in Beispiel 1 beschrieben ausgehend von 2 - Chlor - 5 - chlorsulfonyl - benzoesäure über die 5 - [N - (2 - Butyl) - sulfamoyl] - 2 - chlor - benzoesäure vom Smp. 118—123° und die 5 - [N - (2 - Butyl) - sulfamoyl] - anthranilsäure vom Smp 187—188° erhalten werden. Es schmilzt bei 228—229°.

Beispiel 3

In analoger Weise wie in Beispiel 1 beschrieben erhält man durch Umsetzung von 5 - (N - Cyclohexylsulfamoyl) - isatosäure - anhydrid mit 1-Methylpiperazin das 5 - (N - Cyclohexylsulfamoyl) - anthranilsäure - (4 - methyl) - piperazid vom Smp. 167—168° (aus Aethanol). Das Hydrochlorid schmilzt bei 263—264°.

Das als Ausgangsmaterial zu verwendende 5 - (N - Cyclohexylsulfamoyl) - isatosäureanhydrid kann z.B. in analoger Weise wie in Beispiel 1 beschrieben ausgehend von 2 - Chlor - 5 - chlorsulfonyl - benzoesäure über die 2 - Chlor - 5 - (N - Cyclohexylsulfamoyl) - benzoesäure vom Smp. 168—175° und die 5 - (N - Cyclohexylsulfamoyl)-anthranilsäure vom Smp 204—206° erhalten werden. Es schmilzt bei 232—233°.

Beispiel 4

In analoger Weise wie in Beispiel 1 beschrieben erhält man durch Umsetzung von 5 - [N - (2 - Methylpropyl) - sulfamoyl] - isatosäureanhydrid mit 1-Methylpiperazin das 5 - [N - (2 - Methylpropyl) - sulfamoyl] - anthranilsäure - (4 - methyl) - piperazid vom Smp. 162—163° (aus Aethanol; Smp. des Hydrochlorides: 254—255°).

Das als Ausgangsmaterial zu verwendende 5 - [N - (2 - Methylpropyl) - sulfamoyl] - isatosäureanhydrid kann z.B. in analoger Weise wie in Beispiel 1 beschrieben ausgehend von 2 - Chlor -

5 - chlorsulfonyl - benzoesäure über die 2 - Chlor - 5 - [N - (2 - methylpropyl) - sulfamoyl] - benzoesäure vom Smp. 163—166° und die 5 - [N - (2 - Methylpropyl) - sulfamoyl] - anthranilsäure vom Smp. 203—205° erhalten werden und schmilzt bei 270—273°.

Beispiel 5

In analoger Weise wie in Beispiel 1 beschrieben erhält man durch Umsetzung von 5-(N-Isopropylsulfamoyl)isatosäureanhydrid mit 1-Methylpiperazin das 5 - (N - Isopropylsulfamoyl) - anthranilsäure - (4 - methyl) - piperazid vom Smp. 118—122° (aus Dichlormethan/Diäthyläther; Smp. des Methansulfonates: 232—234°).

Das als Ausgangsmaterial zu verwendende 5 - (N - Isopropylsulfamoyl) - isatosäureanhydrid kann z.B. in analoger Weise wie in Beispiel 1 beschrieben ausgehend von 2-Chlor-5-chlorsulfonyl-benzoesäure über die 2-Chlor-5-(N-isopropylsulfamoyl)-benzoesäure vom Smp. 170—173° und die 5-(N-Isopropylsulfamoyl)-anthranilsäure vom Smp. 230—232° erhalten werden und schmilzt bei 235—237°.

Beispiel 6

In analoger Weise wie in Beispiel 1 beschrieben erhält man durch Umsetzung von 5-(N-Methylsulfamoyl)-isatosäureanhydrid mit 1-Methylpiperazin das 5 - (N - Methylsulfamoyl) - anthranilsäure - (4 - methyl) - piperazid als Oel (Smp. des Methansulfonates: 120—123°).

Das als Ausgangsmaterial zu verwendende 5-(N-Methylsulfamoyl)-isatosäureanhydrid kann z.B. in analoger Weise wie in Beispiel 1 beschrieben ausgehend von 2-Chlor-5-chlorsulfonyl-benzoesäure über die 2-Chlor-5-(N-methylsulfamoyl)-benzoesäure vom Smp. 170—172° und die 5-(N-Methylsulfamoyl)-anthranilsäure vom Smp. 205° erhalten werden und schmilzt bei 260°.

Beispiel 7

In analoger Weise wie in Beispiel 1 beschrieben erhält man durch Umsetzung von 5-(N-Aethylsulfamoyl)-isatosäureanhydrid mit 1-Methylpiperazin das 5-(N-Aethylsulfamoyl)-anthranilsäure-(4-methyl)-piperazid vom Smp. 175—177° (aus Aceton/Aethanol).

Das als Ausgangsmaterial zu verwendende 5-(N-Aethylsulfamoyl)-isatosäureanhydrid kann z.B. in analoger Weise wie in Beispiel 1 beschrieben ausgehend von 2-Chlor-5-chlorsulfonyl-benzoesäure über die 5-(N-Aethylsulfamoyl)-2-chlor-benzoesäure vom Smp. 186—190° und die 5-(N-Aethylsulfamoyl)-anthranilsäure vom Smp. 190—192° erhalten werden und schmilzt bei 253—255°.

Beispiel 8

99 g 5-Methylsulfamoylisatosäureanhydrid werden in 1100 ml Dioxan suspendiert, auf 90° erwärmt und innerhalb von 180 Minuten tropfenweise mit 81 g N-[2-(p-Chlorphenyl)-äthyl]-piperazin, gelöst in 1100 ml Dioxan, versetzt. Man erwärmt auf 90°, bis die Gasentwicklung beendet ist, lässt abkühlen, filtriert und dampft ein. Der Eindampfrückstand wird in Aceton gelöst und über etwa 1300 g Kieselgel (0,06—0,2 mm) filtriert. Nach einem Vorlauf von 600 ml werden die Eluate vereinigt, eingedampft und getrocknet. Man erhält das 5 - (N - Methylsulfamoyl) - anthranilsäure - 4 - [2-(p - chlorphenyl) - äthyl] - piperazid. Dieses kann durch Auflösen in 2000 ml Methanol, Versetzen mit einer Lösung von 25,5 g Fumarsäure in 1000 ml Methanol, Einengen, Kühlen und Abfiltrieren in das Fumarat vom Smp. 224—226° überführt werden.

Das Ausgangsmaterial kann beispielsweise folgendermassen hergestellt werden:

3000 g Chlorsulfonsäure werden innerhalb von etwa 10 Minuten mit 392 g Phosphorpentoxid und anschliessend innerhalb von etwa 60 Minuten mit 835 g Isatosäureanhydrid versetzt, wobei die Innentemperatur auf 40 bis 60° gehalten wird. Man lässt 120 Minuten bei 60° nachrühren, kühlt auf Raumtemperatur ab, giesst vorsichtig auf etwa 20000 g Eis, lässt 30 Minuten nachrühren, nutscht ab, wäscht fünfmal mit je 1 Liter Wasser nach und saugt trocken. Das so erhältliche 5-Chlorsulfonylisatosäureanhydrid kann ohne weitere Reinigung weiterverarbeitet werden.

210 g 5-Chlorsulfonylisatosäureanhydrid werden in 780 ml Aceton suspendiert. In die Suspension wird innerhalb von etwa 50 Minuten 50 g Methylamin eingeleitet, wobei die Innentemperatur durch starkes Kühlen auf 0 bis 5° gehalten wird. Man lässt 3 Stunden bei 0 bis 5° nachrühren, zieht das Aceton unter vermindertem Druck weitgehend ab, versetzt unter Rühren und Kühlung mit 1500 ml Wasser, nutscht ab, wäscht mit Wasser, schlämmt in 500 ml Aethanol auf, saugt erneut ab und trocknet unter vermindertem Druck. Man erhält 5-Methylsulfamoylisatosäureanhydrid vom Smp. 233—234°. Umkristallisieren aus Tetrahydrofuran erhöht den Smp. auf 246—247°.

Beispiel 9

In analoger Weise wie in Beispiel 8 beschrieben erhält man durch Umsetzung von 18,3 g 5-Methylsulfamoylisatosäureanhydrid mit 12,5 g 1-Benzylpiperazin und Chromatographie an Kieselgel mit Chloroform/Aceton (95:5—9:1) und Umsetzung des so erhältlichen 5-(N-Methylsulfamoyl)-anthranilsäure-(4-benzyl)-piperazides mit 5,5 g Fumarsäure dessen Fumarat vom Smp. 220° (Zers.).

Beispiel 10

9,2 g 5-(N-Methylsulfamoyl)-anthranilsäure-(4-benzyl)-piperazid werden in 100 ml Aethanol gelöst, mit 21,6 ml äthanolischer Salzsäure angesäuert, mit 1 g Palladium (5%-ig auf Kohle) versetzt und bei Raumtemperatur und Normaldruck hydriert. Nach Abfiltrieren des Katalysators und Eindampfen erhält man das Hydrochlorid des 5-(N-Methylsulfamoyl)-anthranilsäurepiperazides (Smp. 169°).

Beispiel 11

Tabletten enthaltend 25 mg Wirkstoff, z.B. 1 - [5 - Methylsulfamoyl) - anthraniloyl] - 4 - [2 - (p - chlorphenyl) - äthyl] - piperazin bzw. ein Salz davon können folgendermassen hergestellt werden:

Bestandteile (für 1000 Tabletten)

| | |
|---|---|
| Wirkstoff | 25,0 g |
| Lactose | 100,7 g |
| Weizenstärke | 7,5 g |
| Polyäthylenglykol 6000 | 5,0 g |
| Talkum | 5,0 g |
| Magnesiumstearat | 1,8 g |
| entmineralisiertes Wasser | q.s. |

Herstellung

Sämtliche festen Ingredienzien werden zunächst durch ein Sieb von 0,6 mm Maschenweite getrieben. Dann wird der Wirkstoff, die Lactose, das Talkum, das Magnesiumstearat und die Hälfte der Stärke vermischt. Die andere Hälfte der Stärke wird in 40 ml Wasser suspendiert und diese Suspension zu einer siedenden Lösung des Polyäthylenglykols in 100 ml Wasser hinzugegeben mit der Pulvermischung zu einer knetbaren Masse verarbeitet und das erhaltene Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpresst.

In analoger Weise können auch Tabletten, enthaltend jeweils 25 mg einer anderen der in den Beispielen 1 bis 10 genannten Verbindungen der Formel I hergestellt werden.

**Patentansprüche für die Vertragsstaaten: BE CH DE FR GB IT LU NL SE**

1. Substituierte Anthranilsäureamide der Formel

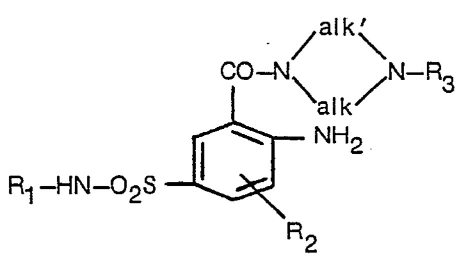

(I),

worin $R_1$ Wasserstoff, Niederalkyl mit bis zu 4 C-Atomen, Niederalkenyl mit bis zu 4 C-Atomen, Cycloalkyl mit 5 bis 8 Ringgliedern, Bicyclo- oder Tricycloalkyl mit jeweils 5 oder 6 Ringgliedern, Cycloalkylniederalkyl mit 5 bis 8 Ringgliedern und mit bis zu 4 C-Atomen im Niederalkylenteil oder Bicyclo- oder Tricycloalkylniederalkyl mit jeweils 5 oder 6 Ringgliedern und mit bis zu 4 C-Atomen im Niederalkylenteil bedeutet, $R_2$ Wasserstoff, Niederalkyl mit bis zu 4 C-Atomen, Niederalkoxy mit bis zu 4 C-Atomen, Halogen bis Atomnummer 35 oder Trifluormethyl bedeutet, alk und alk' gleiches oder verschiedenes, die Stickstoffatome durch 2 C-Atome trennendes Niederalkylen mit bis zu 4 C-Atomen darstellt und $R_3$ Wasserstoff, Niederalkyl mit bis zu 4 C-Atomen, Niederalkenyl mit bis zu 4 C-Atomen, oder einen gegebenenfalls im Phenyl- bzw. Pyridyl- oder Thienylteil durch Niederalkyl mit bis zu 4 C-Atomen, Niederalkoxy mit bis zu 4 C-Atomen, Halogen bis Atomnummer 35 und/oder Trifluormethyl substituierten und im Niederalk(en)ylenteil bis zu 4 C-Atome aufweisenden Phenylniederalkyl-, Phenylniederalkenyl-, Pyridylniederalkyl- oder Thienylniederalkylrest darstellt und ihre Salze.

2. Verbindungen gemäss Anspruch 1, worin $R_1$ Niederalkyl mit bis zu 4 C-Atomen bedeutet, $R_2$ Wasserstoff ist, alk und alk' Aethylen bedeuten und $R_3$ Wasserstoff, Niederalkyl mit bis zu 4 C-Atomen oder einen im Phenylteil gegebenenfalls durch Niederalkyl mit bis zu 4 C-Atomen, Niederalkoxy mit bis zu 4 C-Atomen und/oder Halogen substituierten, bis zu 10 C-Atome aufweisenden Phenylniederalkylrest, bedeutet, und ihre Salze.

3. 5-(N-Methylsulfamoyl)-anthranilsäure-(4-methyl)-piperazid oder ein Salz davon.

4. 5-(N-Methylsulfamoyl)-anthranilsäure-4-[2-(p-chlorphenyl)-äthyl]-piperazid oder ein Salz davon.

5. 5-(N-Methylsulfamoyl)-anthranilsäure-(4-benzyl)-piperazid oder ein Salz davon.

6. 5-(N-Methylsulfamoyl)-anthranilsäurepiperazid oder ein Salz davon.

7. Pharmazeutische Präparate enthaltend eine Verbindung gemäss einem der Ansprüche 1—6 neben üblichen pharmazeutischen Hilfs- und/oder Trägerstoffen.

8. Verfahren zur Herstellung substituierter Anthranilsäureamide der Formel

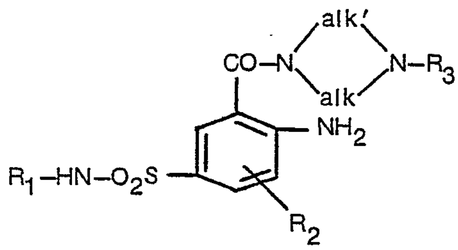

(I),

worin $R_1$ Wasserstoff, Niederalkyl mit bis zu 4 C-Atomen, Niederalkenyl mit bis zu 4 C-Atomen, Cycloalkyl mit 5 bis 8 Ringgliedern, Bicyclo- oder Tricycloalkyl mit jeweils 5 oder 6 Ringgliedern, Cycloalkylniederalkyl mit 5 bis 8 Ringgliedern und mit bis zu 4 C-Atomen in Niederalkylenteil oder Bicyclo- oder Tricycloalkylniederalkyl mit jeweils 5 oder 6 Ringgliedern und mit bis zu 4 C-Atomen im Niederalkylenteil bedeutet, $R_2$ Wasserstoff, Niederalkyl mit bis zu 4 C-Atomen, Niederalkoxy mit bis zu 4 C-Atomen, Halogen bis Atomnummer 35 oder Trifluormethyl bedeutet, alk und alk' gleiches oder verschiedenes, die Stickstoffatome durch 2 C-Atome trennendes Niederalkylen mit bis zu 4 C-Atomen darstellt und $R_3$ Wasserstoff, Niederalkyl mit bis zu 4 C-Atomen, Niederalkenyl mit bis zu 4 C-Atomen, oder einen gegebenenfalls im Phenyl- bzw. Pyridyl- oder Thienylteil durch Niederalkyl mit bis zu 4 C-Atomen, Niederalkoxy mit bis zu 4 C-Atomen, Halogen bis Atomnummer 35 und/oder Trifluormethyl substituierten und im Niederalk(en)ylenteil bis zu 4 C-Atome aufweisenden Phenylniederalkyl-, Phenylniederalkenyl-, Pyridylniederalkyl- oder Thienylniederalkylrest darstellt und ihrer Salze, dadurch gekennzeichnet, dass man in einer Verbindung der Formel

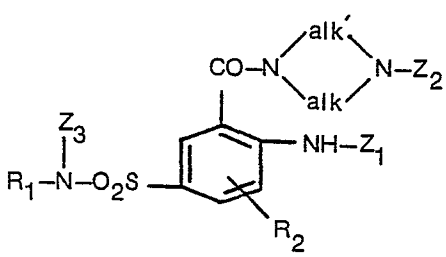

(II)

worin mindestens einer der Reste $Z_1$, $Z_2$ und $Z_3$ einen durch Wasserstoff ersetzbaren Rest Z und ein gegebenenfalls von Z verschiedener Rest $Z_1$, $Z_2$ bzw. $Z_3$ für Wasserstoff oder im Falle eines Restes $Z_2$ für eine von Wasserstoff verschiedene Gruppe $R_3$ steht, den Rest Z durch Wasserstoff ersetzt, oder eine Säure der Formel

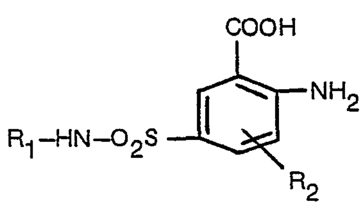

(III)

oder ein reaktionsfähiges funktionelles Derivat mit einem Amin der Formel

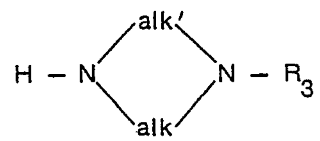

(IV)

oder einem reaktionsfähigen Derivat oder Salz davon umsetzt und gewünschtenfalls die so erhältliche Verbindung in eine andere Verbindung der Formel I umwandelt und/oder eine erhaltene freie Verbindung in ein Salz oder ein erhaltenes Salz in die freie Verbindung oder in ein anderes Salz überführt.

9. Verbindungen der Formel I gemäss einem der Ansprüche 1—6 mit analgetischer, antiinflammatorischer und/oder antiallergischer Wirkung.

10. Verwendung von Verbindungen der Formel I gemäss einem der Ansprüche 1—6 zur Herstellung von Arzneimitteln.

11. Verwendung von Verbindungen der Formel I gemäss einem der Ansprüche 1—6 zur Herstellung von Analgetika, Antiinflammatorika und/oder Antiallergika.

12. Verbindungen der Formel I gemäss einem der Ansprüche 1—6 und 9 zur Anwendung in einem Verfahren zur therapeutischen Behandlung des menschlichen oder Warmblüter-Spezies.

13. Die nach dem Verfahren gemäss Anspruch 8 erhältlichen Verbindungen.

**Patentansprüche für den Vertragsstaat: AT**

1. Verfahren zur Herstellung substituierter Anthranilsäureamide der Formel

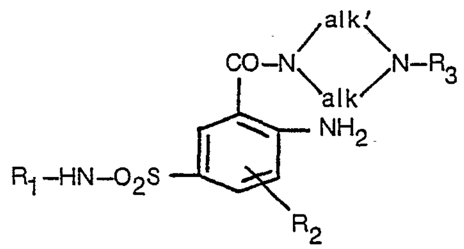

(I),

worin $R_1$ Wasserstoff, Niederalkyl mit bis zu 4 C-Atomen, Niederalkenyl mit bis zu 4 C-Atomen, Cycloalkyl mit 5 bis 8 Ringgliedern, Bicyclo- oder Tricycloalkyl mit jeweils 5 oder 6 Ringgliedern, Cycloalkylniederalkyl mit 5 bis 8 Ringgliedern und mit bis zu 4 C-Atomen im Niederalkylenteil oder Bicyclo- oder Tricycloalkylniederalkyl mit jeweils 5 oder 6 Ringgliedern und mit bis zu 4 C-Atomen im Niederalkylenteil bedeutet, $R_2$ Wasserstoff, Niederalkyl mit bis zu 4 C-Atomen, Niederalkoxy mit bis zu 4 C-Atomen, Halogen bis Atomnummer 35 oder Trifluormethyl bedeutet, alk und alk' gleiches oder verschiedenes, die Stickstoffatome durch 2 C-Atome trennendes Niederalkylen mit bis zu 4 C-Atomen, darstellt und $R_3$ Wasserstoff, Niederalkyl mit bis zu 4 C-Atomen, Niederalkenyl mit bis zu 4 C-Atomen, oder einen gegebenenfalls im Phenyl- bzw. Pyridyl- oder Thienylteil durch Niederalkyl mit bis zu 4 C-Atomen, Niederalkoxy mit bis zu 4 C-Atomen, Halogen bis Atomnummer 35 und/oder Trifluormethyl substituierten und im Niederalk(en)ylenteil bis zu 4 C-Atome aufweisenden Phenylniederalkyl-, Phenylniederalkenyl-, Pyridylniederalkyl- oder Thienylniederalkylrest darstellt und ihre Salze, dadurch gekennzeichnet, dass man in einer Verbindung der Formel

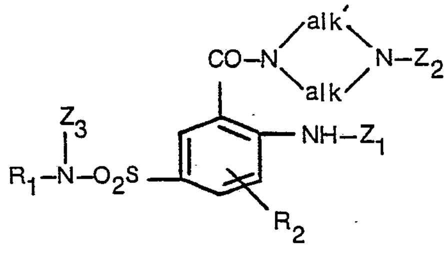

(II)

worin mindestens einer der Reste $Z_1$, $Z_2$ und $Z_3$ einen durch Wasserstoff ersetzbaren Rest Z und ein gegebenenfalls von Z verschiedener Rest $Z_1$, $Z_2$ bzw. $Z_3$ für Wasserstoff oder im Falle eines Restes $Z_2$ für eine von Wasserstoff verschiedene Gruppe $R_3$ steht, den Rest Z durch Wasserstoff ersetzt, oder eine Säure der Formel

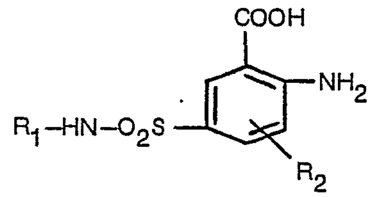

(III)

oder ein reaktionsfähiges funktionelles Derivat davon mit einem Amin der Formel

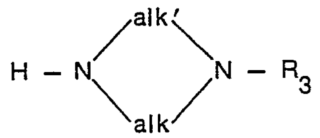

(IV)

oder einem reaktionsfähigen Derivat oder Salz davon umsetzt und gewünschtenfalls die so erhältliche Verbindung in eine andere Verbindung der Formel I umwandelt und/oder eine erhaltene freie Verbindung in ein Salz oder ein erhaltenes Salz in die freie Verbindung oder in ein anderes Salz überführt.

2. Verfahren nach Anspruch 1, dadurch gekennzeichnet, dass man eine Verbindung der allgemeinen Formel I, worin $R_1$ Niederalkyl mit bis zu 4 C-Atomen bedeutet, $R_2$ Wasserstoff ist, alk und alk' Aethylen bedeuten und $R_3$ Wasserstoff, Niederalkyl mit bis zu 4 C-Atomen oder einen im Phenylteil gegebenenfalls durch Niederalkyl mit bis zu 4 C-Atomen, Niederalkoxy mit bis zu 4 C-Atomen und/oder Halogen substituierten, bis zu 10 C-Atomen aufweisenden Phenylniederalkylrest, bedeutet, oder ein Salz davon herstellt.

3. Verfahren nach Anspruch 1, dadurch gekennzeichnet, dass man 5-(N-Methylsulfamoyl)-anthranilsäure-(4-methyl)-piperazid oder ein Salz davon herstellt.

4. Verfahren nach Anspruch 1, dadurch gekennzeichnet, dass man 5-(N-Aethylsulfamoyl)-anthranilsäure-(4-methyl)-piperazid oder ein Salz davon herstellt.

5. Verfahren nach Anspruch 1, dadurch gekennzeichnet, dass man 5-(N-Methylsulfamoyl)-anthranilsäure-4-[2-(p-chlorphenyl)äthyl]-piperazid oder ein Salz davon herstellt.

6. Verfahren nach Anspruch 1, dadurch gekennzeichnet, dass man 5 - (N - Methylsulfamoyl) - anthranilsäure - (4 - benzyl) - piperazid oder ein Salz davon herstellt.

7. Verfahren nach Anspruch 1, dadurch gekennzeichnet, dass man 5-(N-Methylsulfamoyl)-anthranilsäurepiperazid oder ein Salz davon herstellt.

8. Verfahren zur Herstellung eines pharmazeutischen Präparates enthaltend eine Verbindung gemäss einem der Ansprüche 1—7, dadurch gekennzeichnet, dass man diese mit üblichen pharmazeutischen Hilfs- und/oder Trägerstoffen verarbeitet.

**Claims for the Contracting States: BE CH DE FR GB IT LU NL SE**

1. A substituted anthranilamide of the formula

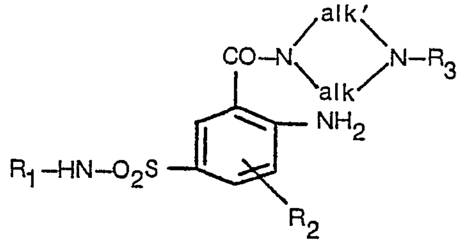

(I),

in which $R_1$ is hydrogen, lower alkyl having not more than 4 C atoms, lower alkenyl having not more than 4 C atoms, cycloalkyl having 5 to 8 ring members, bicyclo- or tricyclo-alkyl having 5 or 6 ring members in each ring, cycloalkyl-lower alkyl having 5 to 8 ring members and having not more than 4 C atoms in the lower alkylene moiety, or bicyclo- or tricyclo-alkyl-lower alkyl having 5 or 6 ring members in each ring and having not more than 4 C atoms in the lower alkylene moiety, $R_2$ is hydrogen, lower alkyl having not more than 4 C atoms, lower alkoxy having not more than 4 C atoms, halogen with an atomic number of not more than 35, or trifluoromethyl, alk and alk' are identical or different lower alkylene which separates the nitrogen atoms by 2 C atoms and has not more than 4 C atoms, and $R_3$ is hydrogen, lower alkyl having not more than 4 C atoms, lower alkenyl having not more than 4 C atoms or a phenyl-lower alkyl radical which can be substituted in the phenyl moiety by lower alkyl having not

more than 4 C atoms, lower alkoxy having not more than 4 C atoms, halogen with an atomic number of not more than 35 and/or trifluoromethyl and has not more than 4 C atoms in the lower alkylene moiety, a phenyl-lower alkenyl radical which can be substituted in the phenyl moiety by lower alkyl having not more than 4 C atoms, lower alkoxy having not more than 4 C atoms, halogen with an atomic number of not more than 35 and/or trifluoromethyl and has not more than 4 C atoms in the lower alkenylene moiety, a pyridyl-lower alkyl radical which can be substituted in the pyridyl moiety by lower alkyl having not more than 4 C atoms, lower alkoxy having not more than 4 C atoms, halogen with an atomic number of not more than 35 and/or trifluoromethyl, and has not more than 4 C atoms in the lower alkylene moiety, or a thienyl-lower alkyl radical, which can be substituted in the thienyl moiety by lower alkyl having not more than 4 C atoms, lower alkoxy having not more than 4 C atoms, halogen with an atomic number of not more than 35, and/or trifluoromethyl and has not more than 4 C atoms in the lower alkylene moiety, or a salt thereof.

2. A compound as claimed in claim 1, in which $R_1$ is lower alkyl having not more than 4 C atoms, $R_2$ is hydrogen, alk and alk′ are ethylene and $R_3$ is hydrogen, lower alkyl having not more than 4 C atoms or a phenyl-lower alkyl radical which can be substituted in the phenyl moiety by lower alkyl having not more than 4 C atoms, lower alkoxy having not more than 4 C atoms and/or halogen, and has not more than 10 C atoms, or a salt thereof.

3. 5-(N-Methylsulfamoyl)-anthranilic acid-(4-methyl)-piperazide or a salt thereof.

4. 5-(N-Methylsulfamoyl)-anthranilic acid-4-[2-(p-chlorophenyl)-ethyl]-piperazide or a salt thereof.

5. 5-(N-Methylsulfamoyl)-anthranilic acid-(4-benzyl)-piperazide or a salt thereof.

6. 5-(N-Methylsulfamoyl)-anthranilic acid piperazide or a salt thereof.

7. A pharmaceutical preparation containing a compound according to any one of claims 1—6, together with customary pharmaceutical auxiliaries and carriers.

8. A process for the manufacture of a substituted anthranilamide of the formula

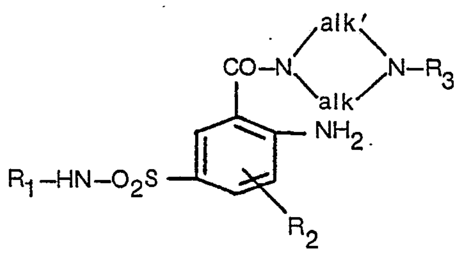

(I),

in which $R_1$ is hydrogen, lower alkyl having not more than 4 C atoms, lower alkenyl having not more than 4 C atoms, cycloalkyl having 5 to 8 ring members, bicyclo- or tricyclo-alkyl having 5 or 6 ring members in each ring, cycloalkyl-lower alkyl having 5 to 8 ring members and having not more than 4 C atoms in the lower alkylène moiety, or bicyclo- or tricyclo-alkyl-lower alkyl having 5 or 6 ring members in each ring and having not more than 4 C atoms in the lower alkylene moiety, $R_2$ is hydrogen, lower alkyl having not more than 4 C atoms, lower alkoxy having not more than 4 C atoms, halogen with an atomic number of not more than 35, or trifluoromethyl, alk and alk′ are identical or different lower alkylene which separates the nitrogen atoms by 2 C atoms and has not more than 4 C atoms, and $R_3$ is hydrogen, lower alkyl having not more than 4 C atoms, lower alkenyl having not more than 4 C atoms or a phenyl-lower alkyl radical which can be substituted in the phenyl moiety by lower alkyl having not more than 4 C atoms, lower alkoxy having not more than 4 C atoms, halogen with an atomic number of not more than 35 and/or trifluoromethyl and has not more than 4 C atoms in the lower alkylene moiety, a phenyl-lower alkenyl radical which can be substituted in the phenyl moiety by lower alkyl having not more than 4 C atoms, lower alkoxy having not more than 4 C atoms, halogen with an atomic number of not more than 35 and/or trifluoromethyl and has not more than 4 C atoms in the lower alkenylene moiety, a pyridyl-lower alkyl radical which can be substituted in the pyridyl moiety by lower alkyl having not more than 4 C atoms, lower alkoxy having not more than 4 C atoms, halogen with an atomic number of not more than 35 and/or trifluoromethyl, and has not more than 4 C atoms in the lower alkylene moiety, or a thienyl-lower alkyl radical, which can be substituted in the thienyl moiety by lower alkyl having not more than 4 C atoms, lower alkoxy having not more than 4 C atoms, halogen with an atomic number of not more than 35, and/or trifluoromethyl and has not more than 4 C atoms in the lower alkylene moiety, or of a salt thereof, which comprises effecting, in a compound of the formula

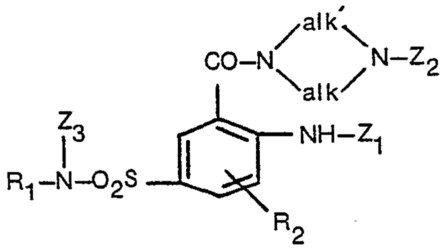

(II)

in which at least one of the radicals $Z_1$, $Z_2$ and $Z_3$ is a radical Z which is replaceable by hydrogen, and a radical $Z_1$, $Z_2$ or $Z_3$ which may differ from Z is hydrogen or, in the case of a radical $Z_2$, is a group $R_3$ which differs from hydrogen, the replacement of the radical Z by hydrogen, or reacting an acid of the formula

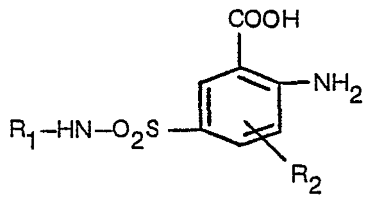

(III)

or a reactive functional derivative thereof, with an amine of the formula

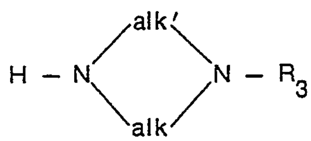

(IV)

or a reactive derivative or salt thereof, and, if desired, converting the compound thus obtainable into another compound of the formula I and/or converting a resulting free compound into a salt and/or converting a resulting salt into the free compound or into another salt.

9. Compounds of the formula I according to any one of claims 1—6 having an analgetic, antiinflammatory and/or antiallergic action.

10. The use of compounds of the formula I according to any one of claims 1—6 for producing pharmaceutical preparations.

11. The use of compounds of the formula I according to any one of claims 1—6 for producing analgetic, antiinflammatory and/or antiallergic preparations.

12. Compounds of the formula I according to any of claims 1—6 for application in a process for the therapeutic treatment of warm-blooded species.

13. The compounds obtainable by the process according to claim 8.

**Claims for the Contracting State: AT**

1. A process for the manufacture of a substituted anthranilamide of the formula

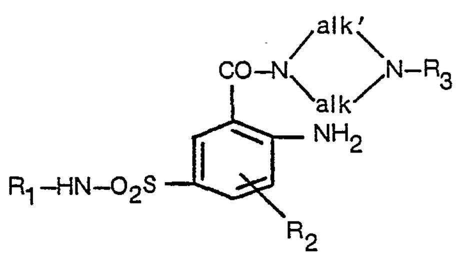

(I),

in which $R_1$ is hydrogen, lower alkyl having not more than 4 C atoms, lower alkenyl having not more than 4 C atoms, cycloalkyl having 5 to 8 ring members, bicyclo- or tricyclo-alkyl having 5 or 6 ring members in each ring, cycloalkyl-lower alkyl having 5 to 8 ring members and having not more than 4 C atoms in the lower alkylene moiety, or bicyclo- or tricyclo-alkyl-lower alkyl having 5 or 6 ring members in each ring and having not more than 4 C atoms in the lower alkylene moiety, $R_2$ is hydrogen, lower

alkyl having not more than 4 C atoms, lower alkoxy having not more than 4 C atoms, halogen with an atomic number of not more than 35, or trifluoromethyl, alk and alk' are identical or different lower alkylene which separates the nitrogen atoms by 2 C atoms and has not more than 4 C atoms, and $R_3$ is hydrogen, lower alkyl having not more than 4 C atoms, lower alkenyl having not more than 4 C atoms or a phenyl-lower alkyl radical which can be substituted in the phenyl moiety by lower alkyl having not more than 4 C atoms, lower alkoxy having not more than 4 C atoms, halogen with an atomic number of not more than 35 and/or trifluoromethyl and has not more than 4 C atoms in the lower alkylene moiety, a phenyl-lower alkenyl radical which can be substituted in the phenyl moiety by lower alkyl having not more than 4 C atoms, lower alkoxy having not more than 4 C atoms, halogen with an atomic number of not more than 35 and/or trifluoromethyl and has not more than 4 C atoms in the lower alkenylene moiety, a pyridyl-lower alkyl radical which can be substituted in the pyridyl moiety by lower alkyl having not more than 4 C atoms, lower alkoxy having not more than 4 C atoms, halogen with an atomic number of not more than 35 and/or trifluoromethyl, and has not more than 4 C atoms in the lower alkylene moiety, or a thienyl-lower alkyl radical, which can be substituted in the thienyl moiety by lower alkyl having not more than 4 C atoms, lower alkoxy having not more than 4 C atoms, halogen with an atomic number of not more than 35, and/or trifluoromethyl and has not more than 4 C atoms in the lower alkylene moiety, or a salt thereof, which comprises effecting, in a compound of the formula

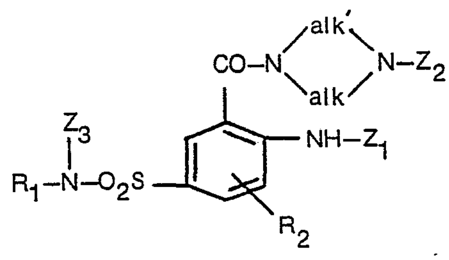

(II)

in which at least one of the radicals $Z_1$, $Z_2$ and $Z_3$ is a radical Z which is replaceable by hydrogen, and a radical $Z_1$, $Z_2$ or $Z_3$ which may differ from Z is hydrogen or, in the case of a radical $Z_2$, is a group $R_3$ which differs from hydrogen, the replacement of the radical Z by hydrogen, or reacting an acid of the formula

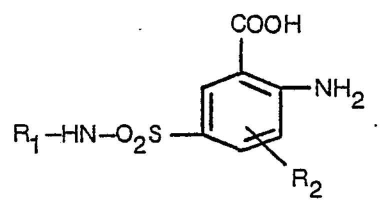

(III)

or a reactive functional derivative thereof, with an amine of the formula

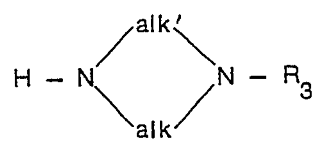

(IV)

or a reactive derivative or salt thereof, and, if desired, converting the compound thus obtainable into another compound of the formula I and/or converting a resulting free compound into a salt and/or converting a resulting salt into the free compound or into another salt.

2. A process according to claim 1 for the manufacture of a compound of the general formula I, in which $R_1$ is lower alkyl having not more than 4 C atoms, $R_2$ is hydrogen, alk and alk' are methylene, and $R_3$ is hydrogen, lower alkyl having not more than 4 C atoms, or a phenyl-lower-alkyl radical which can be substituted in the phenyl moiety by lower alkyl having not more than 4 C atoms, lower alkoxy having not more than 4 C atoms and/or halogen, and which has not more than 10 C atoms, or of a salt thereof.

3. A process according to claim 1, wherein 5-(N-methylsulfamoyl)-anthanilic acid-(4-methyl)-piperazide or a salt thereof is produced.

4. A process according to claim 1, wherein 5-(N-ethylsulfamoyl)-anthranilic acid-(4-methyl)-piperazide or a salt thereof is produced.

5. A process according to claim 1, wherein 5-(N-methylsulfamoyl)-anthranilic acid-4-[2-(p-chlorophenyl)-ethyl]-piperazide or a salt thereof is produced.

6. A process according to claim 1, wherein 5-(N-methylsulfamoyl)-anthranilic acid-(4-benzyl)-piperazide or a salt thereof is produced.

7. A process according to claim 1, wherein 5-(N-methylsulfamoyl)-anthranilic acid piperazide or a salt thereof is produced.

8. A process for manufacturing a pharmaceutical preparation containing a compound according to any one of claims 1—7, in which process the compound is processed together with customary pharmaceutical auxiliaries and/or carriers to make up the preparation.

**Revendications pour les Etats contractants: BE CH DE FR GB IT LU NL SE**

1. Anthranilamides substitués de formule

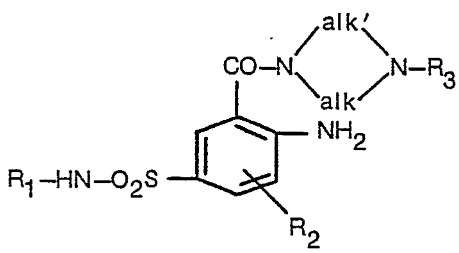

(I),

dans laquelle $R_1$ représente l'hydrogène, un groupe alkyle inférieur contenant jusqu'à 4 atomes de carbone, alcényle inférieur contenant jusqu'à 4 atomes de carbone, cycloalkyle contenant de 5 à 8 chaînons cycliques bicyclo- ou tri-cyclo-alkyle contenant chacun 5 ou 6 chaînons cycliques, cycloalkyl-alkyle inférieur contenant 5 à 8 chaînons cycliques et jusqu'à 4 atomes de carbone dans la partie alkylène inférieur, ou bicyclo- ou tricyclo-alkyl-alkyle inférieur contenant chacun 5 ou 6 chaînons cycliques et jusqu'à 4 atomes de carbone dans la partie alkylène inférieur, $R_2$ représente l'hydrogène, un groupe alkyle inférieur contenant jusqu'à 4 atomes de carbone, alcoxy inférieur contenant jusqu'à 4 atomes de carbone, un halogène de numéro atomique allant jusqu'à 35 ou un groupe trifluorométhyle, alk et alk', ayant des significations identiques ou différentes, représentent un groupe alkylène inférieur contenant jusqu'à 4 atomes de carbone et séparant les atomes d'azote par deux atomes de carbone, et $R_3$ représente l'hydrogène, un groupe alkyle inférieur contenant jusqu'à 4 atomes de carbone, alcényle inférieur contenant jusqu'à 4 atomes de carbone, ou un groupe phényl-alkyle inférieur, phényl-alcényle inférieur, pyridyl-alkyle inférieur ou thiényl-alkyle inférieur éventuellement substitué dans la partie respective phényle, pyridyle ou thiényle par un groupe alkyle inférieur contenant jusqu'à 4 atomes de carbone, alcoxy inférieur contenant jusqu'à 4 atomes de carbone, un halogène de numéro atomique allant jusqu'à 35 et/ou un groupe trifluorométhyle, et contenant jusqu'à 4 atomes de carbone dans la partie alc-(én)-ylène inférieur, et leurs sels.

2. Composés selon la revendication 1, dans lesquels $R_1$ représente un groupe alkyle inférieur contenant jusqu'à 4 atomes de carbone, $R_2$ représente l'hydrogène, alk et alk' représentent des groupes éthylène et $R_3$ représente l'hydrogène, un groupe alkyle inférieur contenant jusqu'à 4 atomes de carbone ou un groupe phényl-alkyle inférieur éventuellement substitué dans la partie phényle par un groupe alkyle inférieur contenant jusqu'à 4 atomes de carbone, alcoxy inférieur contenant jusqu'à 4 atomes de carbone et/ou un halogène, et contenant jusqu'à 10 atomes de carbone, et leurs sels.

3. Le 4-méthyl-pipérazide de l'acide 5-(N-méthylsulfamoyl)-anthranilique ou un sel de ce composé.

4. Le 4-[2-(p-chlorophényl)-éthyl]-pipérazide de l'acide 5-(N-méthylsulfamoyl)-anthranilique ou un sel de ce composé.

5. Le 4-benzyl-pipérazide de l'acide 5-(N-méthylsulfamoyl)-anthranilique ou un sel de ce composé.

6. Le pipérazide de l'acide 5-(N-méthylsulfamoyl)-anthranilique ou un sel de ce composé.

7. Compositions pharmaceutiques contenant un composé selon l'une des revendications 1 à 6 avec des produits auxiliaires et/ou véhicules pharmaceutiques usuels.

8. Procédé de préparation d'anthranilamides substitués de formule

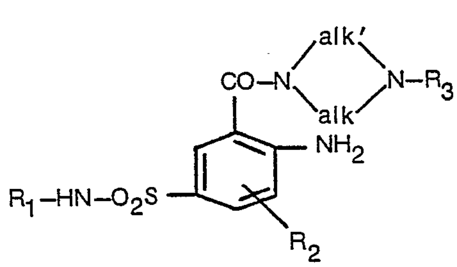

(I),

dans laquelle $R_1$ représente l'hydrogène, un groupe alkyle inférieur contenant jusqu'à 4 atomes de carbone, alcényle inférieur contenant jusqu'à 4 atomes de carbone, cycloalkyle contenant 5 à 8 chaînons cycliques, bicyclo- ou tricyclo-alkyle contenant chacun 5 ou 6 chaînons cycliques, cycloalkyl-alkyle inférieur contenant 5 à 8 chaînons cycliques et jusqu'à 4 atomes de carbone dans la partie alkylène inférieur, ou bicyclo- ou tricyclo-alkyl-alkyle inférieur contenant chacun 5 ou 6 chaînons cycliques et jusqu'à 4 atomes de carbone dans la partie alkylène inférieur, $R_2$ représente l'hydrogène, un groupe alkyle inférieur contenant jusqu'à 4 atomes de carbone, alkoxy inférieur contenant jusqu'à 4 atomes de carbone, un halogène de numéro atomique allant jusqu'à 35 ou un groupe trifluorométhyle, alk et alk', ayant des significations identiques ou différentes, représentent chacun un groupe alkylène inférieur contenant jusqu'à 4 atomes de carbone et séparant les atomes d'azote par deux atomes de carbone, et $R_3$ représente l'hydrogène, un groupe alkyle inférieur contenant jusqu'à 4 atomes de carbone, alcényle inférieur contenant jusqu'à 4 atomes de carbone, ou un groupe phényl-alkyle inférieur, phényl-alcényle inférieur, pyridyl-alkyle inférieur ou thiénylalkyle inférieur éventuellement substitué dans la partie respective phényle, pyridyle ou thiényle par un groupe alkyle inférieur contenant jusqu'à 4 atomes de carbone, alcoxy inférieur contenant jusqu'à 4 atomes, un halogène de numéro atomique allant jusqu'à 35 et/ou un groupe trifluorométhyle, et contenant jusqu'à 4 atomes de carbone dans la partie alc-(én)-ylène inférieur, et de leurs sels, caractérisé en ce que, dans un composé de formule

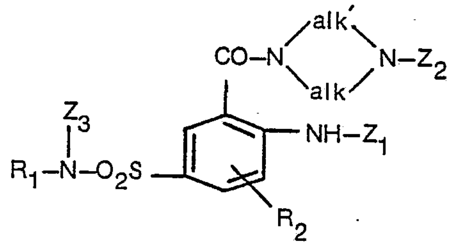

(II)

dans laquelle l'un au moins des restes $Z_1$, $Z_2$ et $Z_3$ est un reste Z remplaçable par l'hydrogène et un reste $Z_1$, $Z_2$ ou $Z_3$ éventuellement différent de Z représente l'hydrogène ou, dans le cas d'un reste $Z_2$, un groupe $R_3$ différent de l'hydrogène, on remplace le reste Z par l'hydrogène, ou bien on fait réagir un acide de formule

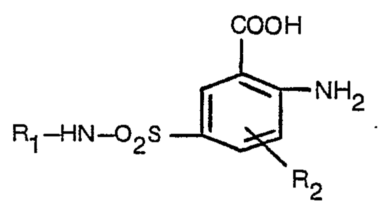

(III)

ou un dérivé fonctionnel réactif d'un tel acide, avec une amine de formule

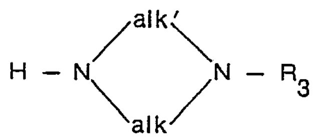

(IV)

ou un dérivé réactif ou un sel de cette amine, et si on le désire, on convertit le composé obtenu en un autre composé de formule I et/ou on convertit un composé libre obtenu en un sel ou un sel obtenu en le composé libre ou en un autre sel.

9. Composés de formule I selon l'une des revendications 1 à 6, possédant une activité analgésique, anti-inflammatoire et/ou anti-allergique.

10. Utilisation des composés de formule I selon l'une des revendications 1 à 6 pour la préparation de médicaments.

11. Utilisation des composés de formule I selon l'une quelconque des revendications 1 à 6, pour la préparation d'analgésiques, d'anti-inflammatoires et/ou d'anti-allergiques.

12. Composés de formule I selon l'une des revendications 1 à 6 et 9 pour l'utilisation dans un procédé pour le traitement thérapeutique de l'homme ou des espèces à sang chaud.

13. Les composés susceptibles d'être obtenus par le procédé selon la revendication 8.

**Revendications pour l'Etat contractant: AT**

1. Procédé de préparation d'anthranilamides substitués de formule

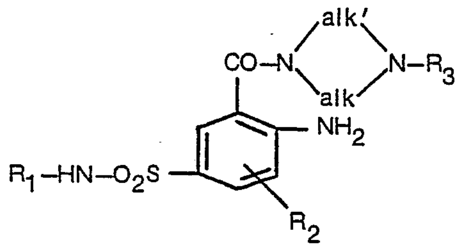

(I),

dans laquelle $R_1$ représente l'hydrogène, un groupe alkyle inférieur contenant jusqu'à 4 atomes de carbone alcényle inférieur contenant jusqu'à 4 atomes de carbone, cycloalkyle contenant 5 à 8 chaînons cycliques, bicyclo- ou tricyclo-alkyle contenant chacun 5 ou 6 chaînons cycliques, cycloalkyl-alkyle inférieur contenant 5 à 8 chaînons cycliques et jusqu'à 4 atomes de carbone dans la partie alkylène inférieur, ou bicyclo ou tricyclo-alkyl-alkyle inférieur contenant chacun 5 ou 6 chaînons cycliques et jusqu'à 4 atomes de carbone dans la partie alkylène inférieur, $R_2$ représente l'hydrogène, un groupe alkyle inférieur contenant jusqu'à 4 atomes de carbone, alcoxy inférieur contenant jusqu' 4 atomes de carbone, un halogène de numéro atomique allant jusqu'à 35 ou un groupe trifluorométhyle, alk et alk', ayant des significations identiques ou différentes, représentent chacun un groupe alkylène inférieur contenant jusqu'à 4 atomes de carbone et séparant les atomes d'azote par deux atomes de carbone, et $R_3$ représente l'hydrogène, un groupe alkyle inférieur contenant jusqu'à 4 atomes de carbone, alcényle inférieur contenant jusqu'à 4 atomes de carbone, ou un groupe phényl-alkyle inférieur, phényl-alcényle inférieur, pyridyl-alkyle inférieur ou thiénylalkyle inférieur éventuellement substitué dans la partie respective phényle, pyridyle ou thiényle par un groupe alkyle inférieur contenant jusqu'à 4 atomes de carbone, alcoxy inférieur contenant jusqu'à 4 atomes, un halogène de numéro atomique allant jusqu'à 35 et/ou un groupe trifluorométhyle, et contenant jusqu'à 4 atomes de carbone dans la partie alc-(én)-ylène inférieur, et de leurs sels, caractérisé en ce que, dans un composé de formule

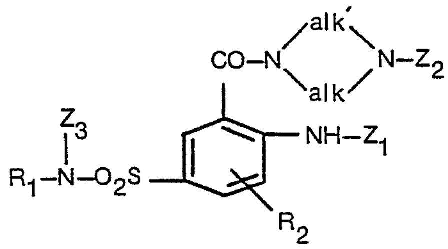

(II)

dans laquelle l'un au moins des restes $Z_1$, $Z_2$ et $Z_3$ est un reste Z remplaçable par l'hydrogène et un reste $Z_1$, $Z_2$ ou $Z_3$ éventuellement différent de Z représente l'hydrogène ou, dans le cas d'un reste $Z_2$, un groupe $R_3$ différent de l'hydrogène, on remplace le reste Z par l'hydrogène, ou bien on fait réagir un acide de formule

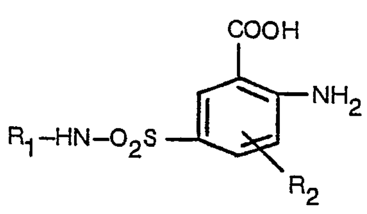

(III)

ou un dérivé fonctionnel réactif d'un tel acide, avec une amine de formule

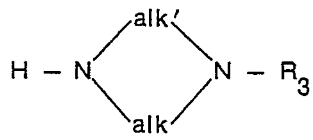

(IV)

ou un dérivé réactif ou un sel de cette amine, et si on le désire, on convertit le composé obtenu en un autre composé de formule I et/ou on convertit un composé libre obtenu en un sel ou un sel obtenu en le composé libre ou en un autre sel.

2. Procédé selon la revendication 1 caractérisé en ce que l'on prépare un composé de formule générale I dans laquelle $R_1$ représente un groupe alkyle inférieur contenant jusqu'à 4 atomes de carbone, $R_2$ représente l'hydrogène, alk et alk' représentent des groupes éthylène et $R_3$ représente l'hydrogène, un groupe alkyle inférieur contenant jusqu'à 4 atomes de carbone ou un groupe phényl-alkyle inférieur éventuellement substitué dans la partie phényle par un groupe alkyle inférieur contenant jusqu'à 4 atomes de carbone, alcoxy inférieur contenant jusqu'à 4 atomes de carbone et/ou un halogène, et contenant jusqu'à 10 atomes de carbone, et leurs sels.

3. Procédé selon la revendication 1 caractérisé en ce que l'on prépare le 4-méthyl-pipérazide de l'acide 5-(N-méthylsulfamoyl)-anthranilique ou un sel de ce composé.

4. Procédé selon la revendication 1, caractérisé en ce que l'on prépare le 4-méthyl-pipérazide de l'acide 5-(N-éthylsulfamoyl)-anthranilique ou un sel de ce composé.

5. Procédé selon la revendication 1, caractérisé en ce que l'on prépare le 4-[2-(p-chlorophényl)-éthyl]-pipérazide de l'acide 5-(N-méthyl-sulfamoyl)-anthranilique ou un sel de ce composé.

6. Procédé selon la revendication 1, caractérisé en ce que l'on prépare le 4-benzyl-pipérazide de l'acide 5-(N-méthylsulfamoyl)-anthranilique ou un sel de ce composé.

7. Procédé selon la revendication 1, caractérisé en ce que l'on prépare le pipérazide de l'acide 5-(N-méthylsulfamoyl)-anthranilique ou un sel de ce composé.

8. Procédé de préparation d'une composition pharmaceutique contenant un composé selon l'une des revendications 1 à 7, caractérisé en ce que l'on met ce composé en oeuvre avec des produits auxiliaires et/ou véhicules pharmaceutiques usuels.